# FASCICULE DE BREVET EUROPEEN

(11) **EP 0 591 373 B1**
(45) Date de publication et mention de la délivrance du brevet: **22.05.1996**
(21) Numéro de dépôt: 92913933.5
(22) Date de dépôt: 25.06.1992
(51) Int. Cl.: A61K 7/13, C07C 217/84

(54) **META-AMINOPHENOLS, LEUR UTILISATION EN TANT QUE COUPLEURS POUR LA TEINTURE D'OXYDATION DES FIBRES KERATINIQUES, COMPOSITIONS ET PROCEDE DE TEINTURE**
META-AMINOPHENOLEN,VERWENDUNG ALS KUPPLUNGEN FÜR OXYDATIONSFARBSTOFFE FÜR KERATINISCHE FÄSER, ZUSAMMENSETZUNGEN UND FARBENPROZESSEN
META-AMINOPHENOLS, THEIR USE AS COUPLERS FOR OXIDATION DYEING OF KERATINOUS FIBRES, DYE COMPOSITIONS AND METHOD

(30) Priorité: 26.06.1991 FR 9107889
(43) Date de publication de la demande: 13.04.1994
(73) Titulaire: L'OREAL, F-75008 Paris (FR)
(72) Inventeur: JUNINO, Alex, F-93190 Livry-Gargan (FR); BONAVENTURE, Nicole, F-94300 Vincennes (FR); LAGRANGE, Alain, F-78400 Chatou (FR)
(74) Mandataire: Casalonga, Axel
(86) Numéro de dépôt international: FR9200580
(87) Numéro de publication internationale: WO9300066

(56) Documents cités:
- EP-A- 0 215 560
- DE-A- 1 617 885
- DE-A- 1 949 749
- FR-A- 2 262 022
- FR-A- 2 348 911
- FR-A- 2 364 888
- GB-A- 2 211 517
- JP-A-81 102 851
- US-A- 3 834 866
- JOURNAL OF THE AMERICAN CHEMICAL SOCIETY, vol. 51, no. 2, Février 1929, GASTON, PA, US pages 569-76; M.T. BOGERT ET. AL.: 'Further Studies of Syringic Acid and its Derivatives'
- CHEMICAL ABSTRACTS, vol. 96, no. 16, 19 Avril 1982, Columbus, Ohio, US, abstract no. 133123w; KONISHIROKU PHOTO INDUSTRY CO.: 'Diffusion Transfer Color Photographic Materials', page 710, colonne 1; & JP-A-81 102 851
- JOURNAL OF THE AMERICAN CHEMICAL SOCIETY Vol. 51, Février 1929 Pages 569-576 BOEGERT et al.
- JP-81102851

## Description

La présente invention a pour objet de nouveaux méta-aminophénols, des compositions tinctoriales pour fibres kératiniques et en particulier pour cheveux humains contenant au moins un méta-aminophénol à titre de coupleur, associé à au moins un précurseur de colorant d'oxydation, ainsi qu'un procédé de teinture mettant en oeuvre lesdites compositions.

Il est usuel, pour la teinture des fibres kératiniques telles que les cheveux humains ou les fourrures, d'employer des compositions tinctoriales contenant des précurseurs de colorants d'oxydation et en particulier des paraphénylènediamines, des orthoaminophénols ou des para-aminophénols que l'on désigne généralement sous le terme de bases d'oxydation.

On sait également que pour faire varier les nuances obtenues avec ces bases d'oxydation, on utilise des modificateurs de coloration ou coupleurs, et en particulier des métaphénylènediamines, des méta-aminophénols et des métadiphénols.

Dans les milieux alcalins oxydants utilisés habituellement en teinture d'oxydation, les paraphénylènediamines et para-aminophénols donnent naissance, en présence de coupleurs tels que les méta-aminophénols, à des indoanilines ou à des indophénols colorés.

Il est important par ailleurs que les bases d'oxydation et les coupleurs, qui sont utilisés dans les compositions de teinture par oxydation, confèrent aux cheveux des colorations stables à la lumière, au lavage, aux intempéries.

Il est nécessaire également que les composés utilisés présentent une bonne innocuité.

On connaît déjà de nombreux coupleurs du type des méta-amino phénols substitués sur le noyau aromatique décrits dans les documents FR-A-2 364 888, GB-A-2 211 517 et US-A-3 834 866. Cependant, un grand nombre d'entre eux ne répondent pas aux exigences souhaitées.

La demanderesse vient de découvrir de nouveaux méta-amino phénols qui allient une très bonne innocuité avec les qualités tinctoriales d'un bon coupleur et qui peuvent donc être utilisés avantageusement comme coupleurs en association avec des précurseurs de colorants d'oxydation, notamment du type para, dans des compositions de teinture d'oxydation pour fibres kératiniques, en milieu alcalin ou acide.

La présente invention concerne une composition tinctoriale d'oxydation, destinée à être utilisée pour la teinture des fibres kératiniques et en particulier des cheveux humains contenant, d'une part, au moins un précurseur de colorant d'oxydation para et/ou ortho et, d'autre part, au moins un méta-aminophénol de formule (I) définie ci-après, à titre de coupleur.

Ainsi, la présente invention concerne des compositions pour la teinture des fibres kératiniques, caractérisée en ce qu'elle contient, dans un milieu approprié pour la teinture :
- au moins un précurseur de colorant d'oxydation du type ortho et ou para, et
- au moins un coupleur de formule : 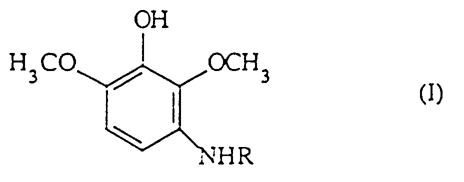
dans laquelle R représente hydrogène, alkyle inférieur en C₁-C₄, monohydroxyalkyle en C₁-C₆; polyhydroxyalkyle en C₂-C₆; ou alkylcarbonyle où les groupes alkyle comportent de 1 à 4 atomes de carbone;
ou leurs sels d'addition avec un acide.

Parmi les méta-aminophénols de formule (I), les composés de formule (II) : 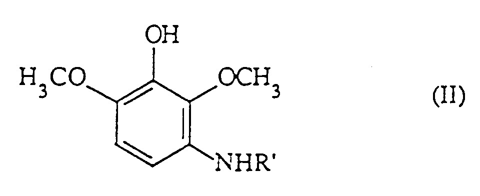
dans laquelle R' représente un radical alkyle en C₁-C₄ ou un radical mono- ou polyhydroxyalkyle en C₂ ou C₃, ainsi que leurs sels d'addition avec un acide, sont nouveaux et constituent un autre objet de l'invention.

L'invention a également pour objet un procédé de teinture des fibres kératiniques mettant en oeuvre les compositions selon l'invention ainsi que des agents de teinture à plusieurs composants, pouvant être sous forme de dispositifs à plusieurs compartiments ou "kit" comprenant les différentes compositions appropriées pour la mise en oeuvre du procédé.

D'autres objets de l'invention apparaîtront à la lecture de la description et des exemples qui suivent.

Dans les définitions ci-dessus, parmi les radicaux alkyle, on peut citer les radicaux méthyle, éthyle, propyle, butyle, isopropyle, isobutyle et tertiobutyle.

Parmi les radicaux monohydroxyalkyle, on peut citer le β-hydroxy éthyle, le β-hydroxypropyle et le γ-hydroxypropyle.

Comme radical polyhydroxyalkyle, on peut citer le β, γ-dihydroxy propyle.

Les sels d'addition avec un acide des méta-aminophénols peuvent être en particulier les sels d'addition avec un acide minéral, tels que l'acide chlorhydrique, bromhydrique ou sulfurique.

Certains composés de formule (I) sont connus. Ainsi, JP-A-81/102 851 décrit le 3-acétylamino 2,6-diméthoxyphénol et le 3-N(tert-butylcarbonyl)amino 2,6-dimethoxyphénol pour leur utilisation dans le domaine photographique. Les composés de formule (I) peuvent être préparés à partir du 3-nitro 2,6-diméthoxyphénol. De plus, la synthèse du composé de formule (I) pour lequel R est H, peut être réalisée selon le procédé décrit dans J.A.C.S., Vol. 51, 1929, page 569.

La synthèse des composés de formule (I) pour lesquels R est différent de H et des composés de formule (II), peut être réalisée à partir du 3-amino 2,6-diméthoxyphénol, suivant des procédés par analogie différents selon la signification des radicaux R ou R'.

Ainsi, la fonction amine peut être alkylée, hydroxyalkylée ou polyhydroxyalkylée selon les procédés classiques d'alkylation, d'hydroxyalkylation ou de polyhydroxyalkylation des amines aromatiques.

A titre d'exemple et selon un premier procédé, pour l'alkylation on peut utiliser, soit des sulfates de dialkyle ou des halogénures d'alkyle tels que l'iodure de méthyle, le bromure d'éthyle, d'isopropyle ou de butyle, et pour l'hydroxyalkylation ou la polyhydroxyalkylation, on peut utiliser des halogénures d'hydroxyalkyles ou de polyhydroxy alkyles.

A titre d'exemple et selon un deuxième procédé, on peut mésyler le 3-amino 2,6-diméthoxyphénol. Le 3-mésylamino 2,6-diméthoxy phénol obtenu est ensuite alkylé, hydroxyalkylé ou polyhydroxyalkylé, puis démésylé.

Pour l'hydroxyalkylation, la méthode préférée consiste à faire réagir le chloroformiate de β-chloroéthyle sur le composé portant la fonction amine, à transformer le carbamate obtenu en oxazolidone qui est ensuite hydrolysée pour conduire au dérivé hydroxyéthylé. Ce procédé est décrit dans la demande de brevet français n° 2 571 364. Le carbamate intermédiaire de β-chloroéthyle peut aussi être soumis directement à l'action d'une base minérale forte, telle que la soude ou la potasse, pour donner le composé (II) dans lequel R' est un radical β-hydroxyéthyle.

Parmi les composés de formule (I), on préfère selon l'invention, les composés suivants :
3-méthylamino 2,6-diméthoxyphénol,
3-éthylamino 2,6-diméthoxyphénol,
3-propylamino 2,6-diméthoxyphénol,
3-butylamino 2,6-diméthoxyphénol,
3-(β-hydroxyéthyl)amino-2,6-diméthoxyphénol,
3-acétylamino 2,6-diméthoxyphénol,
3-(β,γ-dihydroxypropyl)amino 2,6-diméthoxyphénol, et
3-amino 2,6-diméthoxyphénol,
ou leurs sels d'addition avec un acide.

Parmi les composés de formule (I) particulièrement préférés définis ci-dessus, certains répondent également à la formule (II) et sont donc nouveaux.

Il s'agit des
3-méthylamino 2,6-diméthoxyphénol,
3-éthylamino 2,6-diméthoxyphénol,
3-propylamino 2,6-diméthoxyphénol,
3-butylamino 2,6-diméthoxyphénol,
3-(β-hydroxyéthyl)amino 2,6-diméthoxyphénol,
3-(β,γ-dihydroxypropyl)amino 2,6-diméthoxyphénol,
et leurs sels d'addition avec un acide.

Les précurseurs de colorants d'oxydation utilisés selon l'invention, sont du type ortho et/ou para. Ce sont des composés qui ne sont pas des colorants en eux-mêmes, mais qui forment un colorant par un processus de condensation oxydative, soit sur eux-mêmes, soit en présence d'un coupleur ou modificateur.

Ces composés comportent des groupements fonctionnels, soit deux amino, soit un amino et un hydroxy, en position para ou ortho, l'un par rapport à l'autre.

Les précurseurs de type para sont en particulier choisis parmi les paraphénylènediamines, les para-aminophénols, les précurseurs hétérocycliques para, comme la 2,5-diaminopyridine, la 2-hydroxy 5-aminopyridine ou la tétraaminopyrimidine et les bis-phénylène alkylène diamines aussi appelés bases "doubles".

Comme paraphénylènediamines utilisées à titre de précurseurs de colorants d'oxydation selon l'invention, on peut citer les composés répondant à la formule (III) suivante : 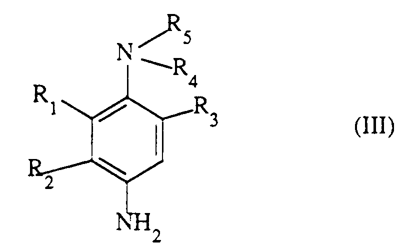
dans laquelle :
R₁, R₂ et R₃, identiques ou différents, représentent un atome d'hydrogène ou d'halogène, un radical alkyle ayant 1 à 4 atomes de carbone, un radical alcoxy ayant 1 à 4 atomes de carbone, un radical carboxy, sulfo, hydroxyalkyle en C₁-C₄;
R₄ et R₅, identiques ou différents, représentent un atome d'hydrogène, un radical alkyle, hydroxyalkyle, alcoxyalkyle, carbamyl alkyle, mésylaminoalkyle, acétylaminoalkyle, uréidoalkyle, carbalcoxy aminoalkyle, sulfoalkyle, pipéridinoalkyle, morpholinoalkyle, phényle éventuellement substitué en para par un groupe amino;
ces groupes alkyle ou alcoxy ayant de 1 à 4 atomes de carbone, ou bien R₄ et R₅ forment, conjointement avec l'atome d'azote auquel ils sont liés, un hétérocycle pipéridino ou morpholino, sous réserve que R₁ ou R₃ représente un atome d'hydrogène lorsque R₄ et R₅ ne représentent pas un atome d'hydrogène, ainsi que leurs sels.

Parmi ces paraphénylènediamines de formule (III), on peut citer les composés suivants : p-phénylènediamine, p-toluylènediamine, méthoxyparaphénylènediamine, chloroparaphénylènediamine, 2,3-diméthylparaphénylènediamine, 2,6-diméthylparaphénylènediamine, 2,6-diéthylparaphénylènediamine, 2,5-diméthylparaphénylènediamine, 2-méthyl 5-méthoxyparaphénylènediamine, 2,6-diméthyl 5-méthoxyparaphénylènediamine, N,N-diméthylparaphénylènediamine, N,N-diéthylparaphénylènediamine, N,N-dipropylparaphénylène diamine, 3-méthyl 4-amino N,N-diéthylaniline, N,N-di(β-hydroxyéthyl)paraphénylènediamine, 3-méthyl 4-amino N,N-di-(β-hydroxyéthyl)aniline, 3-chloro 4-amino N,N-di-(β-hydroxyéthyl)aniline, 4-amino N,N-(éthyl,carbamylméthyl)aniline, 3-méthyl 4-amino N,N-(éthyl,carbamylméthyl)aniline, 4-amino N,N-(éthyl,β-pipéridinoéthyl)aniline, 3-méthyl 4-amino N,N-(éthyl,β-pipéridinoéthyl)aniline, 4-amino N,N-(éthyl,β-morpholinoéthyl) aniline, 3-méthyl 4-amino N,N-(éthyl,β-morpholinoéthyl)aniline, 4-amino N,N-(éthyl,β-acétylaminoéthyl)aniline, 4-amino N-(β-méthoxyéthyl)aniline, 3-méthyl 4-amino N,N-(éthyl,β-acétylamino éthyl)aniline, 4-amino N,N-(éthyl,β-mésylaminoéthyl)aniline, 3-méthyl 4-amino N,N-(éthyl,β-mésylaminoéthyl)aniline, 4-amino N,N-(éthyl,β-sulfoéthyl)aniline, 3-méthyl 4-amino N,N-(éthyl,β-sulfoéthyl) aniline, N- [(4'-amino) phényl]morpholine, N- [(4'-amino) phényl] pipéridine, 2-hydroxyéthylparaphénylènediamine, fluoroparaphénylènediamine, carboxyparaphénylènediamine, sulfoparaphénylène diamine, 2-isopropylparaphénylènediamine, 2-n-propylparaphénylène diamine, hydroxy-2-n-propylparaphénylènediamine, 2-hydroxyméthyl paraphénylènediamine, N,N-diméthyl 3-méthylparaphénylènediamine, N,N-(éthyl,β-hydroxyéthyl)paraphénylènediamine, N-(dihydroxy propyl)paraphénylènediamine, N-4'-aminophénylparaphénylènediamine et N-phénylparaphénylènediamine.

Comme indiqué ci-dessus, ces précurseurs de colorants par oxydation de type para peuvent être introduits dans la composition tinctoriale, soit sous forme de base libre, soit sous forme de sels, tels que chlorhydrate, bromhydrate ou sulfate.

Les précurseurs de colorants de type para peuvent être également choisis parmi les p-aminophénols et lorsque les composés de formules (I) sont utilisés avec des p-aminophénols, ils donnent des nuances particulièrement stables à la lumière, aux intempéries et aux lavages après le développement en présence d'un agent oxydant.

Parmi les p-aminophénols, on peut citer le p-aminophénol, 2-méthyl 4-aminophénol, 3-méthyl 4-aminophénol, 2-chloro 4-aminophénol, 3-chloro 4-aminophénol, 2,6-diméthyl 4-aminophénol, 3,5-diméthyl 4-aminophénol, 2,3-diméthyl 4-aminophénol, 2-hydroxy méthyl 4-aminophénol, 2-(β-hydroxyéthyl)4-aminophénol, 2-méthoxy 4-aminophénol, 3-méthoxy 4-aminophénol, 3-(β-hydroxyéthoxy)4-aminophénol, 2-(β-hydroxyéthoxy)méthyl 4-aminophénol, 2,5-diméthyl 4-aminophénol, 2-méthoxyméthyl 4-aminophénol, 2-éthoxyméthyl 4-aminophénol, 2-aminométhyl 4-aminophénol et 2-β-hydroxyéthylaminométhyl 4-aminophénol.

Comme bis-phénylènealkylènediamine, également appelée base "double", on peut citer les composés répondant à la formule (IV) : 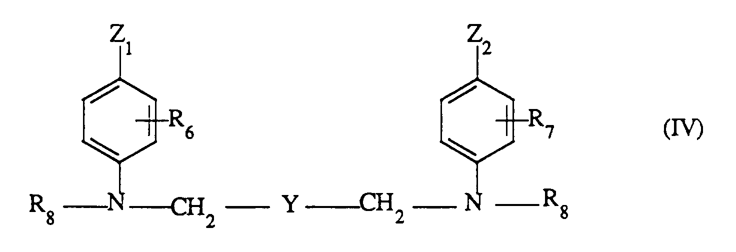
dans laquelle :
Z₁ et Z₂, identiques ou différents, représentent des groupements hydroxyle ou NHR₉, où R₉ désigne un atome d'hydrogène ou un radical alkyle inférieur;
R₆ et R₇, identiques ou différents, représentent, soit des atomes d'hydrogène, soit des atomes d'halogène tels que brome, chlore, fluor, soit encore des groupements alkyle;
R₈ représente un atome d'hydrogène, un groupe alkyle, hydroxy alkyle ou aminoalkyle, dont le reste amino peut être substitué par un ou deux groupements alkyle;
Y représente un radical choisi parmi les radicaux suivants : (CH₂)ₙ-, 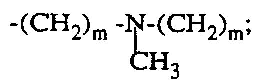
-(CH₂)ₘ-O-(CH₂)ₘ- ; -(CH₂)ₘ-CHOH-(CH₂)ₘ-
n est un nombre entier compris entre O et 8 et m un nombre entier compris entre O et 4, ou leurs sels d'addition avec des acides.

Dans la définition qui précède, les radicaux alkyle ou alcoxy désignent de préférence un groupement ayant 1 à 4 atomes de carbone, et notamment méthyle, éthyle, propyle, méthoxy, éthoxy.

Parmi ces bases doubles, on peut citer les composés choisis parmi le N,N'-bis-(β-hydroxyéthyl)N,N'-bis-(4'-aminophényl) 1,3-diamino 2-propanol, la N,N'-bis(β-hydroxyéthyl)N,N'-bis-(4'-aminophényl)éthylènediamine, la N,N'-bis-(4-aminophényl)tétra méthylènediamine, la N,N'-bis-(β-hydroxyéthyl)N,N'-bis(4-amino phényl)tétraméthylènediamine, la N,N'-bis-(4-méthylaminophényl) tétraméthylènediamine, la N,N'-bis-(éthyl)N,N'-bis-(4'-amino 3'-méthylphényl)éthylènediamine.

On peut également, comme évoqué ci-dessus, utiliser des colorants d'oxydation du type ortho associés avec les coupleurs selon l'invention. Ces colorants peuvent être choisis parmi les ortho-amino phénols comme le 1-amino 2-hydroxybenzène, le 2-amino 5-acétamidophénol, le 6-méthyl 1-hydroxy 2-aminobenzène ou le 4-méthyl 1-amino 2-hydroxybenzène.

On peut utiliser également des orthophénylènediamines.

En plus des coupleurs selon l'invention, les compositions tinctoriales peuvent contenir d'autres coupleurs connus. Parmi ces coupleurs, on peut citer ceux choisis parmi les métadiphénols, les métaaminophénols différents de ceux de formule (I), les métaphénylènediamines, les méta-acylaminophénols, les métauréidophénols, les méta-carbalcoxyaminophénols, l'α-naphtol, les coupleurs possédant un groupement méthylène actif, tels que les composés β-cétoniques, les pyrazolones, les coupleurs hétérocycliques ou les 4-,6-, ou 7-hydroxyindole.

Parmi ces coupleurs, on peut citer particulièrement les coupleurs connus choisis parmi 2,4-dihydroxyphénoxyéthanol, 2,4-dihydroxyanisole, métaaminophénol, résorcine, monométhyléther de résorcine, 2-méthyl résorcine, 2-méthyl 5-N-(β-hydroxyéthyl)aminophénol, 2-méthyl 5-N-(β-mésylaminoéthyl)aminophénol, 6-hydroxybenzomorpholine, 2,4-diaminoanisole, 2,4-diaminophénoxyéthanol, 6-aminobenzomorpholine, [2-N-(β-hydroxyéthyl)amino 4-amino]-phénoxyéthanol, 2-amino 4-N-(β-hydroxyéthyl)aminoanisole, (2,4-diamino)phényl-β,γ-dihydroxypropyléther, 2,4-diaminophénoxyéthylamine, 1,3-diméthoxy 2,4-diaminobenzène, 2-méthyl 5-aminophénol, 2,6-diméthyl 3-aminophénol, 1-amino 3,4-méthylènedioxybenzène, 1-hydroxy 3,4-méthylènedioxybenzène, 2-chloro 6-méthyl 3-aminophénol, 2-méthyl 3-aminophénol, 2-chlororésorcinol, 6-méthoxy 3-hydroxyéthylaminoaniline, 1-éthoxy 2-bis(β-hydroxyéthyl)amino 4-aminobenzène, 3-diéthylaminophénol, 1,3-dihydroxy 2-méthylbenzène, 1-hydroxy 2,4-dichloro 3-aminobenzène, 4,6-hydroxyéthoxy 1,3-diaminobenzène, 4-méthyl 6-éthoxy 1,3-diaminobenzène, 4-chloro 6-méthyl 3-aminophénol, 6-chloro 3-trifluoroéthylaminophénol, 1,3,5-triméthoxy 2,4-diaminobenzène, ou leurs sels.

On peut rajouter à ces compositions, comme cela est bien connu dans l'état de la technique, notamment en vue de nuancer ou d'enrichir en reflets les colorations apportées par les précurseurs de colorants d'oxydation et le coupleur de formules (I), des colorants directs, tels que des colorants azoïques, anthraquinoniques ou les dérivés nitrés de la série benzénique.

L'ensemble des précurseurs de colorants par oxydation de type para et/ou ortho, ainsi que les coupleurs utilisés dans les compositions tinctoriales conformes à l'invention, représente de préférence de 0,1 à 7% en poids par rapport au poids de ladite composition. La concentration en composés (I) peut varier entre 0,05 et 3,5% en poids par rapport au poids total de la composition.

Le milieu approprié pour la teinture est constitué généralement par un milieu aqueux et son pH peut varier entre 4 et 12. Il est ajusté à la valeur désirée à l'aide, soit d'un agent alcalinisant tel que l'ammoniaque, les carbonates alcalins, les alcanolamines comme la mono-, la di- ou la triéthanolamine, soit d'un agent acide comme par exemple l'acide chlorhydrique, l'acide orthophosphorique.

Les compositions tinctoriales conformes à l'invention contiennent également, dans leur forme de réalisation préférée, des agents tensioactifs anioniques, cationiques, non-ioniques, amphotères ou leurs mélanges.

Parmi ces agents tensio-actifs, on peut citer les alkylbenzène sulfonates, les alkylnaphtalènesulfonates, les sulfates, les éther sulfates et les sulfonates d'alcools gras, les sels d'ammonium quaternaires, tels que le bromure de triméthylcétylammonium, le bromure de cétylpyridinium; les éthanolamides d'acides gras éventuellement oxyéthylénés; les acides, les alcools ou les amines polyoxyéthylénés, les alcools polyglycérolés, les alkylphénols polyoxy éthylénés ou polyglycérolés, ainsi que les alkylsulfates polyoxyéthylénés.

Ces agents tensio-actifs sont présents dans les compositions conformes à l'invention dans des proportions comprises entre 0,5 et 40% en poids, et de préférence entre 4 et 30% en poids par rapport au poids total de la composition.

Le milieu peut également être hydroalcoolique. Ces compositions peuvent ainsi également contenir des solvants organiques pour solubiliser les composés qui ne seraient pas suffisamment solubles dans l'eau. Parmi ces solvants, on peut citer à titre d'exemple, les alcanols inférieurs en C₁-C₄, tels que l'éthanol et l'isopropanol; le glycérol; les glycols ou éthers de glycols, comme le 2-butoxyéthanol, l'éthylèneglycol, le propylèneglycol, le monoéthyléther et le monométhyléther du diéthylèneglycol, ainsi que les alcools aromatiques comme l'alcool benzylique ou le phénoxyéthanol et les produits analogues ou leurs mélanges.

Les solvants sont présents de préférence dans des proportions comprises entre 1 et 40% en poids, et en particulier entre 5 et 30% en poids par rapport au poids total de la composition.

Les agents épaissisants que l'on peut ajouter dans les compositions conformes à l'invention peuvent être choisis parmi l'alginate de sodium, la gomme arabique, les dérivés de cellulose, les polymères d'acide acrylique éventuellement réticulés, la gomme de xanthane, les scléroglucanes. On peut également utiliser des agents épaississants minéraux, tels que la bentonite.

Ces agents épaississants sont présents de préférence dans des proportions comprises entre 0,1 et 5% en poids, et en particulier entre 0,5 et 3% en poids par rapport au poids total de la composition.

Les compositions peuvent contenir des agents anti-oxydants choisis en particulier parmi le sulfite de sodium, l'acide thioglycolique, le bisulfite de sodium, l'acide déhydro-ascorbique, l'hydroquinone et l'acide homogentisique. Ces agents antioxydants sont présents dans la composition dans des proportions comprises entre 0,05 et 1,5% en poids par rapport au poids total de la composition.

Ces compositions peuvent également contenir d'autres adjuvants cosmétiquement acceptables, tels que par exemple des agents de pénétration, des agents séquestrants, des parfums, des tampons, etc.

Les compositions conformes à l'invention peuvent se présenter sous des formes diverses, telles que sous forme de liquides, de crèmes, de gels ou sous toute autre forme appropriée pour réaliser une teinture des fibres kératiniques et notamment des cheveux humains. Ces compositions peuvent être conditionnées en flacons aérosols en présence d'un agent propulseur.

Les compositions tinctoriales conformes à l'invention contenant un précurseur de colorant par oxydation du type para et/ou ortho et un coupleur de formules (I) sont utilisées dans les procédés de teinture des fibres kératiniques et en particulier des cheveux humains, suivant un procédé mettant en oeuvre la révélation par un agent oxydant sous forme d'une solution oxydante.

La solution oxydante contient à titre d'agent oxydant, l'eau oxygénée, le peroxyde d'urée, des persels, tels que le persulfate d'ammonium ou des bromates de métaux alcalins. On utilise de préférence une solution d'eau oxygénée à 20 volumes.

Le procédé de teinture des fibres kératiniques et de préférence des fibres kératiniques humaines conforme à l'invention, est essentiellement caractérisé par le fait que l'on applique un composant (A) comportant dans un milieu approprié pour la teinture, au moins un précurseur de colorant d'oxydation du type ortho et/ou para et un composant (B) comportant, dans un milieu approprié pour la teinture, au moins un coupleur de formule (I), et un composant (C) constitué d'une solution oxydante en quantité suffisante pour pouvoir développer une coloration.

Les composants (A), (B) et (C) peuvent être appliqués de façon simultanée ou séquentielle, le composant (A) étant appliqué avant le composant (B) ou en mélange avec celui-ci et le composant (B) étant appliqué avant le composant (C) ou en mélange avec celui-ci.

Selon une première variante, les composants (A), (B) et (C) sont mélangés juste avant l'application sur les cheveux.

Selon une seconde variante, on applique d'abord le mélange des composants (A) et (B) puis le composant (C) dans une seconde étape.

Selon une troisième variante, on applique d'abord le composant (A) puis le mélange des composants (B) et (C).

Enfin, selon une quatrième variante, on applique successivement les composants (A) puis (B) puis (C).

Le pH du mélange du composant (C) avec les composants (A) et (B) ou avec le composant (B), appliqué sur les cheveux, est compris entre 3,5 et 10.

Le procédé de l'invention est mis en oeuvre en prévoyant des temps de pose, pour les différents composants en mélanges appliqués dans chacun des différents temps du procédé, compris entre 5 et 45 minutes, et de préférence de l'ordre de 10 à 30 minutes.

Ainsi, selon la première variante, le mélange obtenu est appliqué sur les cheveux et on laisse poser pendant 5 à 40 minutes, de préférence 10 à 30 minutes, après quoi on rince les cheveux, on les lave au shampooing, on les rince à nouveau et on les sèche.

La présente invention concerne un agent de teinture à plusieurs composants pour des fibres kératiniques, en particulier des cheveux humains, caractérisé par le fait qu'il comporte :
- un composant (A) constitué par une composition contenant, dans un milieu approprié pour la teinture, un précurseur de colorant d'oxydation du type ortho et/ou para;
- un composant (B) constitué par une composition contenant, dans un milieu approprié pour la teinture, un composé de formule (I) ou (II) à titre de coupleur;
- un composant (C) constitué par une solution oxydante,
les composants (A) et (B) pouvant être inclus l'un dans l'autre.

Les exemples ci-après servent à mieux illustrer l'invention, sans pouvoir être considérés comme limitatifs de sa portée.

### EXEMPLES DE PREPARATION

### EXEMPLE 1

### Préparation du 3-(β-hydroxyéthyl)amino 2,6-diméthoxyphénol.

### Stade 1 : préparation du 3-(chloréthoxycarbonyl)amino 2,6-diméthoxy phénol.

On chauffe à 90°C 0,1 mole (20,55 g) de chlorhydrate de 3-amino 2,6-diméthoxyphénol et 11 g de carbonate de calcium dans 62 ml de dioxane.

On coule goutte à goutte 0,11 mole (11,4 ml) de β-chloroéthyl chloroformiate, en 15 minutes.

Après 15 minutes supplémentaires de chauffage, le milieu réactionnel est filtré afin d'éliminer les sels minéraux. Par ajout de 500 ml d'eau au filtrat, on précipite le produit attendu. Il fond à 79°C.

### Stade 2 : préparation de la N-[(3'-hydroxy 2',4'-diméthoxyphényl)] 1,3-oxazolidine, 2-one.

Dans 88 ml d'éthanol, on ajoute 0,079 mole (21,9 g) de 3-(chloréthoxycarbonyl)amino 2,6-diméthoxyphénol préparé à l'étape précédente, puis on coule goutte à goutte 0,175 mode de méthylate de sodium à 30% dans le méthanol. Après dilution à l'eau, puis acidification, le produit attendu précipite. Il fond à 154°C.

### Stade 3 : préparation du 3-(β-hydroxyéthyl)amino 2,6-diméthoxy phénol.

On chauffe au bain marie bouillant 0,064 mole (15,3 g) de N-[(3'-hydroxy 2',4'-diméthoxyphényl)] 1,3-oxazolidine, 2-one préparée à l'étape précédente dans 45 ml de soude 5N. Après 30minutes, le milieu réactionnel refroidi est neutralisé et le produit attendu précipite.

Recristallisé du toluène, il fond à 79°C.

L'analyse du produit obtenu donne les résultats suivants :

| Analyse | Calculé pour C₁₀H₁₅NO₄ | Trouvé |
|---|---|---|
| C | 56,33 | 56,22 |
| H | 7,09 | 7,04 |
| N | 6,57 | 6,58 |
| O | 30,01 | 29,97 |

### EXEMPLE 2

### Préparation du chlorhydrate de 3-méthylamino 2,6-diméthoxyphénol.

### Etape 1 : préparation du 3-N-tosylamino 2,6-diméthoxy 1-tosyloxy benzène.

On ajoute peu à peu à une température de 40°C 0,028 mole (5,32 g) de p-toluènesulfochlorure à une solution de 0,01 mole (2,05 g) de chlorhydrate de 5-amino 2,6-diméthoxyphénol dans la pyridine.

On poursuit l'agitation à 70°C pendant 4 heures après la fin de l'addition.

Le mélange réactionnel est dilué avec de l'eau glacée et par acidification par l'acide chlorhydrique, le produit attendu précipite.

Après essorage et lavage à l'eau, le produit est séché. Il fond à 158°C.

### Etape 2 : Préparation du 3-N,N-tosyl,méthylamino 2,6-diméthoxy 1-tosyloxybenzène.

On dissout 0,0084 mole (4,0 g) de 3-N-tosylamino 2,6-diméthoxy 1-tosyloxybenzène préparé à l'étape précédente dans 12 ml de diméthyl formamide. On ajoute 0,00127 mole (710 mg) de chaux vive puis on élève la température aux environs de 40°C. On introduit alors 0,012 mole (1,1 ml) de sulfate de méthyle. L'addition terminée, on maintient l'agitation pendant 1 heure.

Le produit attendu précipite par dilution du milieu réactionnel avec de l'eau glacée.

Après essorage, lavage à l'eau et acidification par l'acide chlorhydrique, le produit est séché. Il fond à 124°C.

### Etape 3 : préparation du 3-N,N-tosyl,méthylamino 2,6-diméthoxy phénol.

A la température de 90°C, on ajoute peu à peu 0,0081 mole (4,0 g) du composé préparé à l'étape précédente (2) à 80 ml de soude 7,5N. On ajoute ensuite de l'éthanol jusqu'à dissolution totale.

La température de reflux est maintenue pendant 15 minutes après la fin de l'addition.

Le milieu réactionnel est dilué par un mélange glace/eau. Le produit attendu qui a précipité est essoré, lavé à l'eau et séché sous vide en présence d'anhydride phosphorique. Il fond à 110°C.

### Etape 4 : Préparation du 3-méthylamino 2,6-diméthoxyphénol.

On ajoute peu à peu à une température de 80°C 4 ml d'acide chlorhydrique à une solution de 2,3 g (0,0068 mole) du produit préparé à l'étape précédente (3) dans 4 ml d'acide acétique.

La température est maintenue à 110°C pendant 3 heures après la fin de l'addition.

Le milieu réactionnel est dilué avec de l'eau puis neutralisé avec de l'ammoniaque jusqu'à pH=6,7, puis extrait à l'acétate d'éthyle.

Les phases acétate d'éthyle sont lavées à l'eau, séchées sur sulfate de soude puis évaporées à sec.

Par ajout d'éthanol chlorhydrique, on précipite le chlorhydrate de 3-méthylamino 2,6-diméthoxyphénol qui est recristallisé de l'isopropanol.

L'analyse RMN du proton est conforme à la structure attendue.

### EXEMPLE DE TEINTURE 1

On prépare le mélange tinctorial suivant :

| | |
|---|---|
| - Chlorhydrate de 3-amino 2,6-diméthoxyphénol | 0,514 g |
| - p-phénylènediamine | 0,27 g |
| - Alcool oléique polyglycérolé à 2 moles de glycérol | 4,5 g |
| - Alcool oléique polyglycérolé à 4 moles de glycérol | 4,5 g |
| - Oleylamine oxyéthylénée à 12 moles d'oxyde d'éthylène, vendue sous la dénomination "ETHOMEEN O 12" par la Société ARMOON HESS CHEMICAL Ltd | 4,5 g |
| - Diéthanolamide de coprah vendu sous la dénomination "COMPERLAN KD" par la Société HENKEL | 9,0 g |
| - Propylèneglycol | 4,0 g |
| - 2-butoxyéthanol | 8,0 g |
| - Ethanol à 96°C | 6,0 g |
| - Sel pentasodique de l'acide diéthylène triamine pentacétique vendu sous la dénomination "MASQUOL DTPA" par la Société PROTEX | 2,0 g |
| - Hydroquinone | 0,15 g |
| - Solution de bisulfite de sodium à 35°Bé | 1,3 g |
| - Ammoniaque à 22°Bé | 10,0 g |
| - pH = 10 | |
| - Eau qsp | 100,0 g |

Au moment de l'emploi, on ajoute 100 g d'eau oxygénée à 20 volumes. Le mélange, appliqué pendant 20 minutes à 35°C sur des cheveux naturellement blancs à 90% leur confère, après shampooing et rinçage, une coloration brun légèrement rouge.

Les degrés Baumé (Bé) sont définis au Merck Index.

### EXEMPLE DE TEINTURE 2

On prépare le mélange tinctorial suivant :

| | |
|---|---|
| - Chlorhydrate de 3-amino 2,6-diméthoxyphénol | 0,514 g |
| - p-aminophénol | 0,272 g |
| - Alcool oléique polyglycérolé à 2 moles de glycérol | 4,5 g |
| - Alcool oléique polyglycérolé à 4 moles de glycérol | 4,5 g |
| - Oleylamine oxyéthylénée à 12 moles d'oxyde d'éthylène, vendue sous la dénomination "ETHOMEEN O 12" par la Société ARMOON HESS CHEMICAL Ltd | 4,5 g |
| - Diéthanolamide de coprah vendu sous la dénomination "COMPERLAN KD" par la Société HENKEL | 9,0 g |
| - Propylèneglycol | 4,0 g |
| - 2-butoxyéthanol | 8,0 g |
| - Ethanol à 96°C | 6,0 g |
| - Sel pentasodique de l'acide diéthylène triamine pentacétique, vendu sous la dénomination "MASQUOL DTPA" par la Société PROTEX | 2,0 g |
| - Hydroquinone | 0,15 g |
| - Solution de bisulfite de sodium à 35°Bé | 1,3 g |
| - Ammoniaque à 22°Bé | 10,0 g |
| - pH = 10 | |
| - Eau qsp | 100,0 g |

Au moment de l'emploi, on ajoute 100 g d'eau oxygénée à 20 volumes. Le mélange, appliqué pendant 20 minutes à 30°C sur des cheveux naturellement blancs à 90% leur confère, après shampooing et rinçage, une coloration brun clair.

### EXEMPLE DE TEINTURE 3

On prépare le mélange tinctorial suivant :

| | |
|---|---|
| - Chlorhydrate de 3-amino 2,6-diméthoxyphénol | 0,61 g |
| - Dichlorhydrate de p-phénylènediamine | 0,627 g |
| - Alcool oléique polyglycérolé à 2 moles de glycérol | 4,0 g |
| - Alcool oléique polyglycérolé à 4 moles de glycérol à 78% de MA | 5,69 g MA |
| - Acide oléique | 3,0 g |
| - Amine oléique à 2 moles d'oxyde d'éthylène, vendue sous la dénomination "ETHOMEEN O 12" par la Société AKZO | 7,0 g |
| - Laurylamino succinamate de diéthylaminopropyle, sel de sodium à 55% de MA | 3,0 g MA |
| - Alcool oléique | 5,0 g |
| - Diéthanolamide d'acide oléique | 12,0 g |
| - Propylèneglycol | 3,5 g |
| - Alcool éthylique | 7,0 g |
| - Dipropylèneglycol | 0,5 g |
| - Monométhyléther de propylèneglycol | 9,0 g |
| - Métabisulfite de sodium en solution aqueuse à 35% de MA | 0,45 g MA |
| - Acétate d'ammonium | 0,8 g |
| - Antioxydant, séquestrant qs | |
| - Parfum, conservateurs qs | |
| - Monoéthanolamine qsp pH = 9,8 | |
| - Eau déminéralisée qsp | 100,0 g |

Au moment de l'emploi, on ajoute 100 g d'eau oxygénée à 20 volumes dont le pH est ajusté entre 1 et 1,5 avec de l'acide orthophosphorique pur à 85%. Le mélange dont le pH est d'environ 6,5, appliqué pendant 30 minutes sur des cheveux naturellement blancs à 90% leur confère, après shampooing et rinçage, une coloration cendré irisé.

### EXEMPLE DE TEINTURE 4

On prépare le mélange tinctorial suivant :

| | |
|---|---|
| - 3-(β-hydroxyéthyl)amino 2,6-diméthoxyphénol | 0,53 g |
| - Sulfate, hydrate de N,N-di (β-hydroxyéthyl)paraphénylènediamine | 0,78 g |
| - Alcool oléique polyglycérolé à 2 moles de glycérol | 4,5 g |
| - Alcool oléique polyglycérolé à 4 moles de glycérol | 4,5 g |
| - Oleylamine oxyéthylénée à 12 moles d'oxyde d'éthylène, vendue sous la dénomination "ETHOMEEN O 12" par la Société ARMOON HESS CHEMICAL Ltd | 4,5 g |
| - Diéthanolamide de coprah vendu sous la dénomination "COMPERLAN KD" par la Société HENKEL | 9,0 g |
| - Propylèneglycol | 4,0 g |
| - 2-butoxyéthanol | 8,0 g |
| - Ethanol à 96°C | 6,0 g |
| - Sel pentasodique de l'acide diéthylène triamine pentacétique, vendu sous la dénomination "MASQUOL DTPA" par la Société PROTEX | 2,0 g |
| - Hydroquinone | 0,15 g |
| - Solution de bisulfite de sodium à 35°Bé | 1,3 g |
| - Ammoniaque à 22°Bé | 10,0 g |
| - pH = 10,2 | |
| - Eau qsp | 100,0 g |

Au moment de l'emploi, on ajoute 100 g d'eau oxygénée à 20 volumes. Le mélange, appliqué pendant 20 minutes à 37°C sur des cheveux naturellement blancs à 90% leur confère, après shampooing et rinçage, une coloration gris bleu légèrement rouge.

### EXEMPLE DE TEINTURE 5

On prépare le mélange tinctorial suivant :

| | |
|---|---|
| - Chlorhydrate de 3-méthylamino 2,6-diméthoxy phénol | 0,55 g |
| - 2-méthoxyméthyl 4-aminophénol | 0,38 g |
| - Hydroxyéthyl cellulose vendue sous la dénomination "CELLOSIZE WP 03" par la Société UNION CARBIDE | 2,0 g |
| - Laurylsulfate d'ammonium | 5,0 g |
| - 2-butoxyéthanol | 15,0 g |
| - Ethanol à 96°C | 6,0 g |
| - Ammoniaque à 22°Bé | 10,0 g |
| - Solution de bisulfite de sodium à 35°Bé | 1,5 g |
| - Hydroquinone | 0,15 g |
| - pH = 10,1 | |
| - Eau qsp | 100,0 g |

Au moment de l'emploi, on ajoute 100 g d'eau oxygénée à 20 volumes. Le mélange, appliqué pendant 25 minutes à 37°C sur des cheveux décolorés leur confère, après shampooing et rinçage, une coloration beige vert.

### EXEMPLE DE TEINTURE 6

On prépare un mélange tinctorial suivant :

| | |
|---|---|
| - 3-(β-hydroxyéthyl)amino 2,6-diméthoxy phénol | 0,53 g |
| - Dichlorhydrate de 4-amino N-(β-méthoxyéthyl)aniline | 0,59 g |
| - Hydroxyéthylcellulose vendue sous la dénomination "CELLOSIZE WP 03" par la Société UNION CARBIDE | 2,0 g |
| - Laurylsulfate d'ammonium | 5,0 g |
| - 2-butoxyéthanol | 15,0 g |
| - Ethanol à 96°C | 5,0 g |
| - Sel pentasodique de l'acide diéthylène triamine pentacétique, vendu sous la dénomination "MASQUOL DTPA" par la Société PROTEX | 2,0 g |
| - Monoéthanolamine qsp pH = 9 | |
| - Eau qsp | 100,0 g |

Au moment de l'emploi, on ajoute 100 g d'eau oxygénée à 20 volumes dont le pH est ajusté entre 1 et 1,5 par de l'acide orthophosphorique. Le pH final est alors de 6,8.

Le mélange, appliqué pendant 20 minutes à 37°C sur des cheveux naturellement blancs à 90% leur confère, après shampooing et rinçage, une coloration gris anthracite clair.

### EXEMPLE DE TEINTURE 7

| | |
|---|---|
| - Paraphénylènediamine | 0,07 g |
| - 3-(β-hydroxyéthylamino)2,6-diméthoxyphénol | 0,058 g |
| - Dichlorhydrate de 3-amino 6-(β-hydroxyéthoxy)aniline | 0,027 g |
| - Métaaminophénol | 0,05 g |
| - Résorcine | 0,1 g |
| - 2-nitro 6-méthyl 3-aminophénol | 0,015 g |
| - Alcool cétylstéarylique vendu sous la dénomination "ALFOL 1618" par la Société CONDEA | 8,0 g |
| - Cétylstéarylsulfate de sodium vendu sous la dénomination "CIRE DE LANETTE E" par la Société HENKEL | 0,5 g |
| - Huile de ricin éthoxylée vendue sous la dénomination "CEMULSOL B" par la Société RHONE POULENC | 1,0 g |
| - Diéthanolamide oléique | 1,5 g |
| - Sel pentasodique de l'acide diéthylène triamine pentacétique vendu sous la dénomination "MASQUOL DTPA" par la Société PROTEX | 2,5 g |
| - Ammoniaque à 22°Bé | 11,0 g |
| - Eau qsp | 100,0 g |
| - pH = 10 | |

Au moment de l'emploi, on ajoute 100 g d'eau oxygénée à 20 volumes. Le mélange appliqué 25 minutes à 37°C sur des cheveux naturellement blancs leur confère, après shampooing et rinçage, une coloration gris bleuté.

### EXEMPLE DE TEINTURE 8

| | |
|---|---|
| - 3-(β-hydroxyéthylamino)2,6-diméthoxyphénol | 0,3 g |
| - 3-amino 6-méthylphénol | 0,7 g |
| - Dichlorhydrate de 2-méthylparaphénylènediamine | 0,3 g |
| - 3-méthyl 4-aminophénol | 0,7 g |
| - Nonylphénol oxyéthyléné à 4 moles d'oxyde d'éthylène vendu sous la dénomination "CEMULSOL NP4" par la Société RHONE POULENC | 12,0 g |
| - Nonylphénol oxyéthyléné à 9 moles d'oxyde d'éthylène vendu sous la dénomination "CEMULSOL NP9" par la Société RHONE POULENC | 15,0 g |
| - Alcool oléique polyglycérolé à 2 moles de glycérol | 1,5 g |
| - Alcool oléique polyglycérolé à 4 moles de glycérol | 1,5 g |
| - Propylèneglycol | 6,0 g |
| - Acide éthylène diamine tétracétique | 0,12 g |
| - Ammoniaque à 22°Bé | 11,0 g |
| - Eau qsp | 100,0 g |
| - pH = 9,4 | |

Au moment de l'emploi, on ajoute 100 g d'eau oxygénée à 20 volumes. Le mélange, appliqué 25 minutes à 37°C sur des cheveux décolorés leur confère, après shampooing et rinçage, une coloration lie de vin.

## Revendications

1. Composition pour la teinture des fibres kératiniques, caractérisée en ce qu'elle comporte, dans un milieu approprié pour la teinture, au moins
- un précurseur de colorant d'oxydation du type ortho et ou para choisi parmi les paraaminophénols, les précurseurs hétérocycliques du type para, les bis-phénylènealkylènediamines, et les paraphénylène diamines de formule (III) : 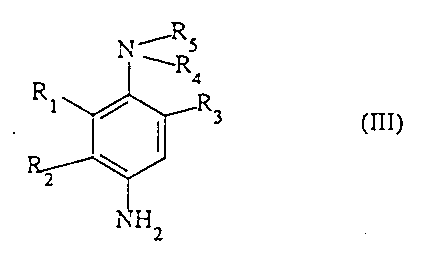 dans laquelle :
R₁, R₂ et R₃, identiques ou différents, représentent un atome d'hydrogène ou d'halogène, un radical alkyle ayant 1 à 4 atomes de carbone, un radical alcoxy ayant 1 à 4 atomes de carbone, un radical carboxy, sulfo, hydroxyalkyle en C₁-C₄;
R₄ et R₅, identiques ou différents, représentent un atome d'hydrogène, un radical alkyle, hydroxyalkyle, alcoxyalkyle, carbamyl alkyle, mésylaminoalkyle, acétylaminoalkyle, uréidoalkyle, carbalcoxy aminoalkyle, sulfoalkyle, pipéridinoalkyle, morpholinoalkyle, phényle éventuellement substitué en para par un groupe amino;
ces groupes alkyle ou alcoxy ayant de 1 à 4 atomes de carbone, ou bien R₄ et R₅ forment, conjointement avec l'atome d'azote auquel ils sont liés, un hétérocycle pipéridino ou morpholino, sous réserve que R₁ ou R₃ représente un atome d'hydrogène lorsque R₄ et R₅ ne représentent pas un atome d'hydrogène, ou leurs sels,
- au moins un coupleur de formule : 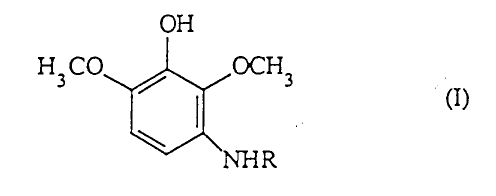 dans laquelle R représente hydrogène, alkyle inférieur en C₁-C₄, monohydroxyalkyle en C₁-C₆; polyhydroxyalkyle en C₂-C₆; ou alkylcarbonyle où les groupes alkyle comportent de 1 à 4 atomes de carbone;
ou leurs sels d'addition avec un acide.

2. Composition selon la revendication 1, caractérisée en ce que les composés de formule (I) sont choisis parmi :
3-méthylamino 2,6-diméthoxyphénol,
3-éthylamino 2,6-diméthoxyphénol,
3-propylamino 2,6-diméthoxyphénol,
3-butylamino 2,6-diméthoxyphénol,
3-(β-hydroxyéthyl)amino-2,6-diméthoxyphénol,
3-acétylamino 2,6-diméthoxyphénol,
3-(β,γ-dihydroxypropyl)amino 2,6-diméthoxyphénol, et
3-amino 2,6-diméthoxyphénol.

3. Composition selon l'une des revendications 1 et 2, caractérisée en ce qu'elle comporte, à titre de précurseur de colorant d'oxydation, au moins un composé de formule (III) choisi parmi :
p-phénylènediamine, p-toluylènediamine, méthoxyparaphénylène diamine, chloroparaphénylènediamine, 2,3-diméthylparaphénylène diamine, 2,6-diméthylparaphénylènediamine, 2,6-diéthylparaphénylènediamine, 2,5-diméthylparaphénylènediamine, 2-méthyl 5-méthoxyparaphénylènediamine, 2,6-diméthyl 5-méthoxyparaphénylène diamine, N,N-diméthylparaphénylènediamine, N,N-diéthylparaphénylènediamine, N,N-dipropylparaphénylènediamine, 3-méthyl 4-amino N,N-diéthylaniline, N,N-di(β-hydroxyéthyl)paraphénylène diamine, 3-méthyl 4-amino N,N-di-(β-hydroxyéthyl)aniline, 3-chloro 4-amino N,N-di-(β-hydroxyéthyl)aniline, 4-amino N,N-(éthyl, carbamylméthyl)aniline, 3-méthyl 4-amino N,N-(éthyl, carbamylméthyl)aniline, 4-amino N,N-(éthyl,β-pipéridinoéthyl)aniline, 3-méthyl 4-amino N,N-(éthyl,β-pipéridinoéthyl)aniline, 4-amino N,N-(éthyl,β-morpholinoéthyl)aniline, 3-méthyl 4-amino N,N-(éthyl,β-morpholinoéthyl)aniline, 4-amino N,N-(éthyl,β-acétylaminoéthyl)aniline, 4-amino N-(β-méthoxyéthyl)aniline, 3-méthyl 4-amino N,N-(éthyl,β-acétylaminoéthyl)aniline, 4-amino N,N-(éthyl,β-mésylaminoéthyl)aniline, 3-méthyl 4-amino N,N-(éthyl,β-mésylaminoéthyl)aniline, 4-amino N,N-(éthyl,β-sulfoéthyl)aniline, 3-méthyl 4-amino N,N-(éthyl,β-sulfoéthyl)aniline, N-[(4'-amino)phényl]morpholine, N-[(4'-amino)phényl]pipéridine, 2-hydroxyéthylparaphénylènediamine, fluoroparaphénylènediamine, carboxyparaphénylènediamine, sulfoparaphénylènediamine, 2-isopropylparaphénylènediamine, 2-n-propylparaphénylènediamine, hydroxy-2-n-propylparaphénylènediamine, 2-hydroxyméthylparaphénylènediamine, N,N-diméthyl 3-méthylparaphénylènediamine, N,N-(éthyl,β-hydroxyéthyl)paraphénylènediamine, N-(dihydroxypropyl) paraphénylènediamine, N-4'-aminophénylparaphénylènediamine, N-phénylparaphénylènediamine.

4. Composition selon l'une des revendications 1 à 3, caractérisée en ce qu'elle comporte, à titre de précurseur de colorant d'oxydation du type para, au moins un paraaminophénol choisi parmi :
p-aminophénol, 2-méthyl 4-aminophénol, 3-méthyl 4-aminophénol, 2-chloro 4-aminophénol, 3-chloro 4-aminophénol, 2,6-diméthyl 4-aminophénol, 3,5-diméthyl 4-aminophénol, 2,3-diméthyl 4-aminophénol, 2-hydroxyméthyl 4-aminophénol, 2-(β-hydroxyéthyl)4-aminophénol, 2-méthoxy 4-aminophénol, 3-(β-hydroxyéthoxy)4-aminophénol, 2-(β-hydroxyéthoxy)méthyl 4-aminophénol, 2-éthoxy méthyl 4-aminophénol, 3-méthoxy 4-aminophénol, 2,5-diméthyl 4-aminophénol, 2-méthoxyméthyl 4-aminophénol, 2-aminométhyl 4-aminophénol et 2-β-hydroxyéthylaminométhyl 4-aminophénol.

5. Composition selon l'une des revendications 1 à 4, caractérisée en ce qu'elle comporte, à titre de précurseur de colorant d'oxydation, au moins une bis-phénylène alkylène diamine de formule (IV) : 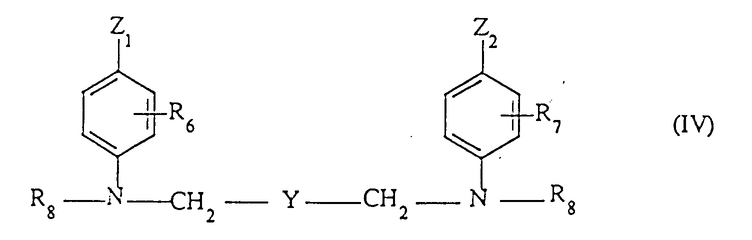 dans laquelle :
Z₁ et Z₂, identiques ou différents, représentent des groupements hydroxyle ou NHR₉, où R₉ désigne un atome d'hydrogène ou un radical alkyle inférieur;
R₆ et R₇, identiques ou différents, représentent, soit des atomes d'hydrogène, soit des atomes d'halogène, soit encore des groupements alkyle;
R₈ représente un atome d'hydrogène, un groupe alkyle, hydroxy alkyle ou aminoalkyle, dont le reste amino peut être substitué par un ou deux groupements alkyle;
Y représente un radical choisi parmi les radicaux suivants : (CH₂)ₙ-, 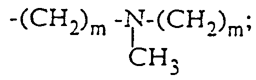 -(CH₂)ₘ-O-(CH₂)ₘ- ; -(CH₂)ₘ-CHOH-(CH₂)ₘ-
n est un nombre entier compris entre O et 8 et m un nombre entier compris entre O et 4, ou leurs sels d'addition avec des acides.

6. Composition selon la revendication 5, caractérisée en ce qu'elle comporte au moins une bis-phénylène alkylène diamine choisie parmi : le N,N'-bis-(β-hydroxyéthyl)N,N'-bis-(4'-aminophényl)1,3-diamino 2-propanol, la N,N'-bis(β-hydroxyéthyl)N,N'-bis-(4'-aminophényl) éthylènediamine, la N,N'-bis-(4-aminophényl)tétraméthylènediamine, la N,N'-bis-(β-hydroxyéthyl)N,N'-bis(4-aminophényl)tétraméthylène diamine, la N,N'-bis-(4-méthylaminophényl) tétraméthylènediamine, la N,N'-bis-(éthyl)N,N'-bis-(4'-amino 3'- méthylphényl)éthylènediamine.

7. Composition selon l'une des revendications 1 à 6, caractérisée en ce qu'elle comporte, à titre de précurseur de colorant d'oxydation du type ortho, au moins un orthoaminophénol et/ou une orthophénylènediamine ou leurs sels d'addition avec un acide.

8. Composition selon l'une des revendications 1 à 7, caractérisée en ce qu'elle comporte, en outre, au moins un autre coupleur choisi parmi :
les métadiphénols, les métaaminophénols différents de ceux de formule (I), les métaphénylènediamines, les métaacylaminophénols, les métauréidophénols, les métacarbalcoxyaminophénols, l'α-naphtol, les coupleurs possédant un groupement méthylène actif, tels que les composés β-cétoniques, les pyrazolones, les coupleurs hétérocycliques ou les 4-,6-, ou 7-hydroxyindole.

9. Composition selon l'une des revendications 1 à 8, caractérisée en ce qu'elle comporte au moins un coupleur choisi parmi :
2,4-dihydroxyphénoxyéthanol, 2,4-dihydroxyanisole, métaaminophénol, résorcine, 2-méthylrésorcine, monométhyléther de résorcine, 2-méthyl 5-N-(β-hydroxyéthyl)aminophénol, 2-méthyl 5-N-(β-mésylaminoéthyl)aminophénol, 6-hydroxybenzomorpholine, 2,4-diaminoanisole, 2,4-diaminophénoxyéthanol, 6-aminobenzomorpholine, [2-N-(β-hydroxyéthyl)amino 4-amino]-phénoxyéthanol, 2-amino 4-N-(β-hydroxyéthyl)aminoanisole, (2,4-diamino)phényl-β,γ-dihydroxypropyléther, 2,4-diaminophénoxyéthylamine, 1,3-diméthoxy 2,4-diaminobenzène, 2-méthyl 5-aminophénol, 2,6-diméthyl 3-aminophénol, 1-amino 3,4-méthylènedioxybenzène, 1-hydroxy 3,4-methylènedioxybenzène, 2-chloro 6-méthyl 3-aminophénol, 2-méthyl 3-aminophénol, 2-chlororésorcinol, 6-méthoxy 3-hydroxyéthylaminoaniline, 1-éthoxy 2-bis(β-hydroxyéthyl)amino 4-aminobenzène, 3-diéthylaminophénol, 1,3-dihydroxy 2-méthylbenzène, 1-hydroxy 2,4-dichloro 3-aminobenzène, 4,6-hydroxyéthoxy 1,3-diaminobenzène, 4-méthyl 6-éthoxy 1,3-diaminobenzène, 4-chloro 6-méthyl 3-aminophénol, 6-chloro 3-trifluoroéthylaminophénol, 2-méthyl 5-aminophenol, 1,3,5-triméthoxy 2,4-diaminobenzène, ou leurs sels.

10. Composition selon l'une des revendications 1 à 9, caractérisée en ce que les précurseurs de colorant d'oxydation du type para et/ou ortho, avec les coupleurs, représentent 0,1 à 7% en poids par rapport au poids total de la composition.

11. Composition selon l'une des revendications 1 à 10, caractérisée en ce que la concentration en composés de formules (I) est de 0,05 à 3,5% en poids par rapport au poids total de la composition.

12. Composition selon l'une des revendications 1 à 11, caractérisée en ce qu'elle comporte en outre un ou plusieurs composants choisis parmi les agents alcalinisants, les agents tensioactifs, les solvants organiques, les agents épaississants, les agents anti-oxydants, les agents de pénétration, les agents séquestrants, les parfums et les tampons.

13. Composition selon l'une des revendications 1 à 12, caractérisée en ce que son pH est compris entre 4 et 12.

14. Composé de formule (II) : 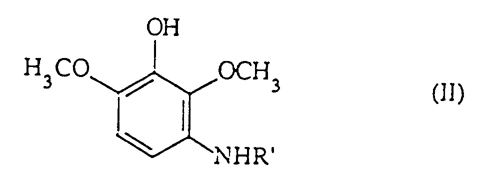 dans laquelle R' représente un radical alkyle en C₁-C₄ ou un radical mono- ou polyhydroxyalkyle en C₂ ou C₃, et ses sels d'addition avec un acide.

15. Composé selon la revendication 14, choisi parmi 3-méthyl amino 2,6-diméthoxyphénol, 3-éthylamino 2,6-diméthoxyphénol, 3-propylamino 2,6-diméthoxyphénol, 3-butylamino 2,6-diméthoxy phénol, 3-(β-hydroxyéthyl)amino 2,6-diméthoxyphénol, 3-(β,γ-dihydroxypropyl)amino 2,6-diméthoxyphénol.

16. Procédé de teinture des fibres kératiniques, caractérisé par le fait que l'on applique :
un composant (A) comportant dans un milieu approprié pour la teinture, au moins un précurseur de colorant d'oxydation du type ortho et/ou para, tel que défini à l'une des revendications précédentes, et
un composant (B) comportant, dans un milieu approprié pour la teinture, au moins un coupleur de formule (I) : 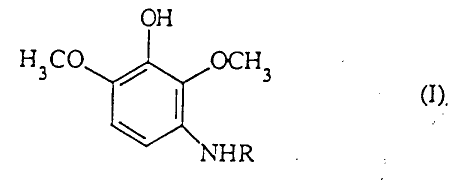 dans laquelle R représente hydrogène, alkyle inférieur en C₁-C₄, monohydroxyalkyle en C₁-C₆; polyhydroxyalkyle en C₂-C₆; ou alkylcarbonyle où les groupes alkyle comportent de 1 à 4 atomes de carbone;
ou ses sels d'addition avec un acide;
et un composant (C) constitué d'une solution oxydante en quantité suffisante pour pouvoir développer une coloration;
les composants (A), (B) et (C) étant appliqués de façon simultanée ou séquentielle, le composant (A) étant appliqué avant le composant (B) ou en mélange avec celui-ci et le composant (B) étant appliqué avant le composant (C) ou en mélange avec celui-ci.

17. Procédé selon la revendication 16, caractérisé en ce que le pH du mélange du composant (C) avec les composants (A) et (B) ou avec le composant (B) est compris entre 3,5 et 10.

18. Agent de teinture à plusieurs composants pour des fibres kératiniques, en particulier des cheveux humains, caractérisé par le fait qu'il comporte :
- un composant (A) constitué par une composition contenant, dans un milieu approprié pour la teinture, au moins un précurseur de colorant d'oxydation du type ortho et/ou para tel que défini dans les revendications précédentes; et
- un composant (B) constitué par une composition contenant, dans un milieu approprié pour la teinture, un composé de formule (I) : 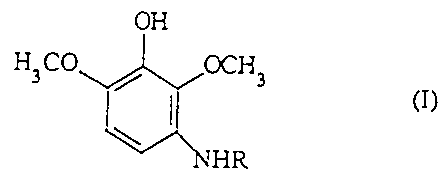 dans laquelle R représente hydrogène, alkyle inférieur en C₁-C₄, monohydroxyalkyle en C₁-C₆; polyhydroxyalkyle en C₂-C₆; ou alkylcarbonyle où les groupes alkyle comportent de 1 à 4 atomes de carbone;
ou ses sels d'addition d'acide, à titre de coupleur; et
- un composant (C) constitué par une solution oxydante, les composants (A) et (B) pouvant être inclus l'un dans l'autre.

## Claims

1. Composition for the dyeing of keratinous fibres, characterized in that it contains, in a medium suitable for dyeing, at least
- one ortho and/or para type oxidation dye precursor chosen from para-aminophenols, para type heterocyclic precursors, bis(phenylene)alkylenediamines and the para-phenylenediamines of formula (III): 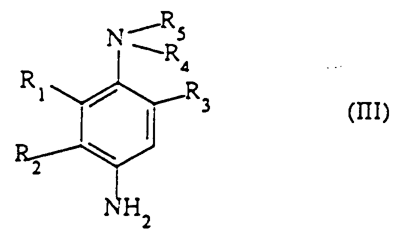 in which:
R₁, R₂ and R₃, which may be identical or different, represent a hydrogen or halogen atom, an alkyl radical having 1 to 4 carbon atoms, an alkoxy radical having 1 to 4 carbon atoms, a carboxyl, sulpho, C₁-C₄ hydroxyalkyl radical;
R₄ and R₅, which may be identical or different, represent a hydrogen atom, an alkyl, hydroxyalkyl, alkoxyalkyl, carbamylalkyl, mesylaminoalkyl, acetylaminoalkyl, ureidoalkyl, carbalkoxyaminoalkyl, sulphoalkyl, piperidinoalkyl, morpholinoalkyl radical, a phenyl radical optionally substituted at the para position with an amino group;
these alkyl or alkoxy groups having from 1 to 4 carbon atoms, or alternatively R₄ and R₅, together with the nitrogen atom to which they are linked, form a piperidino or morpholino heterocycle, with the proviso that R₁ or R₃ represents a hydrogen atom when R₄ and R₅ do not represent a hydrogen atom, or their salts,
- at least one coupler of formula: 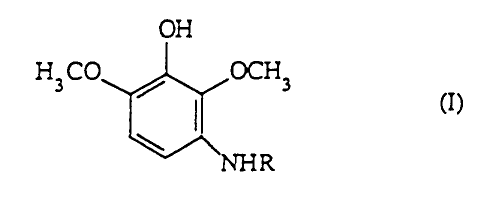 in which R represents hydrogen, C₁-C₄ lower alkyl, C₁-C₆ monohydroxyalkyl; C₂-C₆ polyhydroxyalkyl; or alkylcarbonyl in which the alkyl groups contain from 1 to 4 carbon atoms;
or their addition salts with an acid.

2. Composition according to Claim 1, characterized in that the compounds of formula (I) are chosen from:
3-methylamino-2,6-dimethoxyphenol,
3-ethylamino-2,6-dimethoxyphenol,
3-propylamino-2,6-dimethoxyphenol,
3-butylamino-2,6-dimethoxyphenol,
3-(β-hydroxyethyl)amino-2,6-dimethoxyphenol,
3-acetylamino-2,6-dimethoxyphenol,
3-(β,γ-dihydroxypropyl)amino-2,6-dimethoxyphenol,
and
3-amino-2,6-dimethoxyphenol.

3. Composition according to one of Claims 1 and 2, characterized in that it contains, as oxidation dye precursor, at least one compound of formula (III) chosen from:
p-phenylenediamine, p-toluylenediamine, methoxy-para-phenylenediamine, chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, 2-methyl-5-methoxy-para-phenylenediamine, 2,6-dimethyl-5-methoxy-para-phenylenediamine, N,N-dimethyl-para-phenylenediamine, N,N-diethyl-para-phenylenediamine, N,N-dipropyl-para-phenylenediamine, 3-methyl-4-amino-N,N-diethylaniline, N,N-di(β-hydroxyethyl)-para-phenylenediamine, 3-methyl-4-amino-N,N-di(β-hydroxyethyl)aniline, 3-chloro-4-amino-N,N-di(β-hydroxyethyl)aniline, 4-amino-N,N-(ethyl,carbamylmethyl)aniline, 3-methyl-4-amino-N,N-(ethyl,carbamylmethyl)aniline, 4-amino-N,N-(ethyl,β-piperidinoethyl)aniline, 3-methyl-4-amino-N,N-(ethyl,β-piperidinoethyl)aniline, 4-amino-N,N-(ethyl, β-morpholinoethyl)aniline, 3-methyl-4-amino-N,N-(ethyl,β-morpholinoethyl)aniline, 4-amino-N,N-(ethyl, β-acetylaminoethyl)aniline, 4-amino-N-(β-methoxyethyl)aniline, 3-methyl-4-amino-N,N-(ethyl,β-acetylaminoethyl)aniline, 4-amino-N,N-(ethyl,β-mesylaminoethyl)aniline, 3-methyl-4-amino-N,N-(ethyl,β-mesylaminoethyl)aniline, 4-amino-N,N-(ethyl,β-sulphoethyl)aniline, 3-methyl-4-amino-N,N-(ethyl,β-sulphoethyl)aniline, N-[(4'-amino)phenyl]morpholine, N-[(4'-amino)phenyl]piperidine, 2-hydroxyethyl-para-phenylenediamine, fluoro-para-phenylenediamine, carboxy-para-phenylenediamine, sulpho-para-phenylenediamine, 2-isopropyl-para-phenylenediamine, 2-n-propyl-para-phenylenediamine, hydroxy-2-n-propyl-para-phenylenediamine, 2-hydroxymethyl-para-phenylenediamine, N,N-dimethyl-3-methyl-para-phenylenediamine, N,N-(ethyl,β-hydroxyethyl)-para-phenylenediamine, N-(dihydroxypropyl)-para-phenylenediamine, N-4'-aminophenyl-para-phenylenediamine, N-phenyl-para-phenylenediamine.

4. Composition according to one of Claims 1 to 3, characterized in that it contains, as para type oxidation dye precursor, at least one para-aminophenol chosen from:
p-aminophenol, 2-methyl-4-aminophenol, 3-methyl-4-aminophenol, 2-chloro-4-aminophenol, 3-chloro-4-aminophenol, 2,6-dimethyl-4-aminophenol, 3,5-dimethyl-4-aminophenol, 2,3-dimethyl-4-aminophenol, 2-hydroxymethyl-4-aminophenol, 2-(β-hydroxyethyl)-4-aminophenol, 2-methoxy-4-aminophenol, 3-(β-hydroxyethoxy)-4-aminophenol, 2-(β-hydroxyethoxy)methyl-4-aminophenol, 2-ethoxymethyl-4-aminophenol, 3-methoxy-4-aminophenol, 2,5-dimethyl-4-aminophenol, 2-methoxymethyl-4-aminophenol, 2-aminomethyl-4-aminophenol and 2-β-hydroxyethylaminomethyl-4-aminophenol [sic].

5. Composition according to one of Claims 1 to 4, characterized in that it contains, as oxidation dye precursor, at least one bis(phenylene)alkylenediamine of formula (IV): 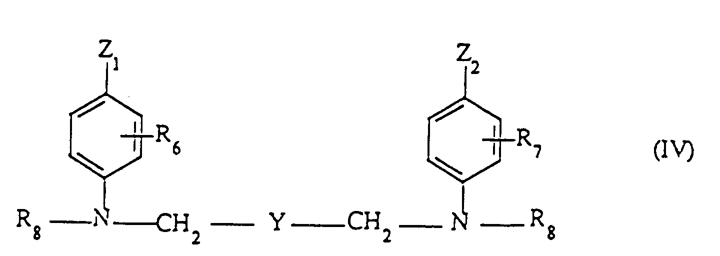 in which:
Z₁ and Z₂, which may be identical or different, represent hydroxyl groups or groups NHR₉, where R₉ denotes a hydrogen atom or a lower alkyl radical;
R₆ and R₇, which may be identical or different, represent either hydrogen atoms or halogen atoms or alternatively alkyl groups;
R₈ represents a hydrogen atom, an alkyl or hydroxyalkyl group or an aminoalkyl group in which the amino residue may be substituted with one or two alkyl groups;
Y represents a radical chosen from the following radicals:
(CH₂)ₙ-, 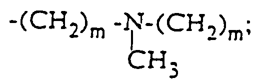 -(CH₂)ₘ-O-(CH₂)ₘ- ; -(CH₂)ₘ-CHOH-(CH₂)ₘ-
n is an integer between 0 and 8 and m, an integer between 0 and 4, or their addition salts with acids.

6. Composition according to Claim 5, characterized in that it contains at least one bis(phenylene)alkylenediamine chosen from: N,N'-bis(β-hydroxyethyl)-N,N'-bis-(4'-aminophenyl)-1,3-diamino-2-propanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)tetramethylenediamine, N,N'-bis(4-methylaminophenyl)tetramethylenediamine, N,N'-bis(ethyl)-N,N'-bis(4'-amino-3'-methylphenyl)ethylenediamine.

7. Composition according to one of Claims 1 to 6, characterized in that it contains, as ortho type oxidation dye precursor, at least one ortho-aminophenol and/or one ortho-phenylenediamine or their addition salts with an acid.

8. Composition according to one of Claims 1 to 7, characterized in that it contains, in addition, at least one other coupler chosen from:
meta-diphenols, meta-aminophenols other than those of formula (I), meta-phenylenediamines, meta-acylaminophenols, meta-ureidophenols, meta-carbalkoxyaminophenols, α-naphthol, couplers possessing an active methylene group, such as β-keto compounds, pyrazolones heterocyclic couplers or 4-, 6- or 7-hydroxyindole.

9. Composition according to one of Claims 1 to 8, characterized in that it contains at least one coupler chosen from:
2,4-dihydroxyphenoxyethanol, 2,4-dihydroxyanisole, meta-aminophenol, resorcinol, 2-methylresorcinol, resorcinol monomethyl ether, 2-methyl-5-N-(β-hydroxyethyl)aminophenol, 2-methyl-5-N-(β-mesylaminoethyl)aminophenol, 6-hydroxybenzomorpholine, 2,4-diaminoanisole, 2,4-diaminophenoxyethanol, 6-aminobenzomorpholine, [2-N-(β-hydroxyethyl)amino-4-amino]phenoxyethanol [sic], 2-amino-4-N-(β-hydroxyethyl)aminoanisole, (2,4-diamino)phenyl [sic] β,γ-dihydroxypropyl ether, 2,4-diaminophenoxyethylamine, 1,3-dimethoxy-2,4-diaminobenzene, 2-methyl-5-aminophenol, 2,6-dimethyl-3-aminophenol, 1-amino-3,4-methylenedioxybenzene, 1-hydroxy-3,4-methylenedioxybenzene, 2-chloro-6-methyl-3-aminophenol, 2-methyl-3-aminophenol, 2-chlororesorcinol, 6-methoxy-3-hydroxyethylaminoaniline, 1-ethoxy-2-bis(β-hydroxyethyl)amino-4-aminobenzene, 3-diethylaminophenol, 1,3-dihydroxy-2-methylbenzene, 1-hydroxy-2,4-dichloro-3-aminobenzene, 4,6-hydroxyethoxy-1,3-diaminobenzene, 4-methyl-6-ethoxy-1,3-diaminobenzene, 4-chloro-6-methyl-3-aminophenol, 6-chloro-3-trifluoroethylaminophenol, 2-methyl-5-aminophenol [sic], 1,3,5-trimethoxy-2,4-diaminobenzene or their salts.

10. Composition according to one of Claims 1 to 9, characterized in that the para and/or ortho type oxidation dye precursors, with the couplers, represent 0.1 to 7% by weight relative to the total weight of the composition.

11. Composition according to one of Claims 1 to 10, characterized in that the concentration of compounds of formulae (I) [sic] is from 0.05 to 3.5% by weight relative to the total weight of the composition.

12. Composition according to one of Claims 1 to 11, characterized in that it contains, in addition, one or more components chosen from alkalinizing agents, surfactants, organic solvents, thickening agents, anti-oxidants, penetrating agents, sequestering agents, perfumes and buffers.

13. Composition according to one of Claims 1 to 12, characterized in that its pH is between 4 and 12.

14. Compound of formula (II): 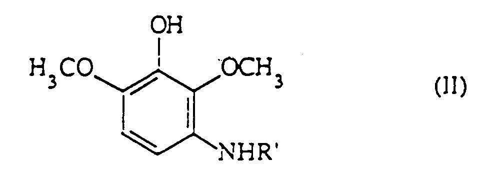 in which R' represents a C₁-C₄ alkyl radical or a C₂ or C₃ mono- or polyhydroxyalkyl radical, and its addition salts with an acid.

15. Compound according to Claim 14, chosen from 3-methylamino-2,6-dimethoxyphenol, 3-ethylamino-2,6-dimethoxyphenol, 3-propylamino-2,6-dimethoxyphenol, 3-butylamino-2,6-dimethoxyphenol, 3-(β-hydroxyethyl)amino-2,6-dimethoxyphenol, 3-(β,γ-dihydroxypropyl)amino-2,6-dimethoxyphenol.

16. Process for dyeing keratinous fibres, characterized in that there are applied:
a component (A) containing, in a medium suitable for dyeing, at least one ortho and/or para type oxidation dye precursor as defined in one of the preceding claims, and
a component (B) containing, in a medium suitable for dyeing, at least one coupler of formula (I): 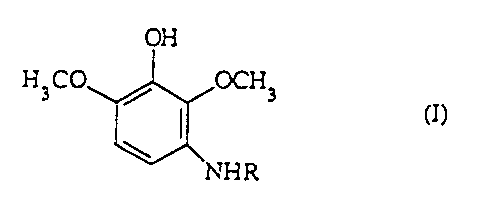 in which R represents hydrogen, C₁-C₄ lower alkyl, C₁-C₆ monohydroxyalkyl; C₂-C₆ polyhydroxyalkyl; or alkylcarbonyl in which the alkyl groups contain from 1 to 4 carbon atoms;
or its addition salts with an acid;
and a component (C) consisting of an oxidizing solution in a sufficient amount to be able to develop a colouration;
the components (A), (B) and (C) being applied simultaneously or sequentially, the component (A) being applied before the component (B) or mixed with the latter, and the component (B) being applied before the component (C) or mixed with the latter.

17. Process according to Claim 16, characterized in that the pH of the mixture of the component (C) with the components (A) and (B) or with the component (B) is between 3.5 and 10.

18. Multi-component dyeing agent for keratinous fibres, especially human hair, characterized in that it contains:
- a component (A) consisting of a composition containing, in a medium suitable for dyeing, at least one ortho and/or para type oxidation dye precursor as defined in the preceding claims; and
- a component (B) consisting of a composition containing, in a medium suitable for dyeing, a compound of formula (I): 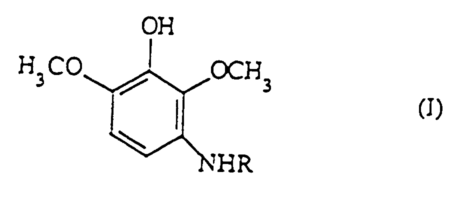 in which R represents hydrogen, C₁-C₄ lower alkyl, C₁-C₆ monohydroxyalkyl; C₂-C₆ polyhydroxyalkyl; or alkylcarbonyl in which the alkyl groups contain from 1 to 4 carbon atoms;
or its addition salts with an acid, as coupler; and
- a component (C) consisting of an oxidizing solution,
it being possible for one of the components (A) and (B) to be included in the other.

## Patentansprüche

1. Zusammensetzung zur Färbung keratinischer Fasern,
dadurch **gekennzeichnet**, daß
sie, in einem zur Färbung geeigneten Milieu, mindestens
- eine Oxidationsfarbstoff-Vorstufenverbindung vom o- und/oder p-Typ, ausgewählt aus p-Aminophenolen, heterozyklischen Vorstufenverbindungen des p-Typs, Bisphenylenalkylendiaminen und aus p-Phenylendiaminen der Formel (III): 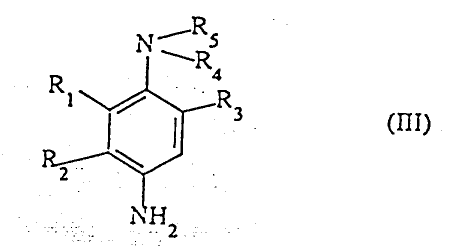 worin gilt:
R₁, R₂ und R₃ stellen, gleich oder verschieden, ein Wasserstoff- oder Halogenatom, einen Alkylrest mit 1 bis 4 Kohlenstofatomen, einen Alkoxyrest mit 1 bis 4 Kohlenstoffatomen, einen Carboxy-, Sulfo- oder einen C₁₋₄-Hydroxyalkylrest dar;
R₄ und R₅ stellen, gleich oder verschieden, ein Wasserstoffatom, einen Alkyl-, Hydroxyalkyl-, Alkoxyalkyl-, Carbamylalkyl-, Mesylaminoalkyl-, Acetylaminoalkyl-, Ureidoalkyl-, Carbalkoxyaminoalkyl-, Sulfoalkyl-, Piperidinoalkyl-, Morpholinoalkyl- oder einen Phenylrest dar, der in p-Position mit einer Aminogruppe gegebenenfalls substituiert ist,
wobei diese Alkyl- oder Alkoxygruppen 1 bis 4 Kohlenstoffatome aufweisen, oder R₄ und R₅ bilden, zusammen mit dem Stickstoffatom, an das sie gebunden sind, einen Piperidino- oder Morpholino-Heterozyklus, mit der Maßgabe, daß R₁ oder R₃ ein Wasserstoffatom darstellen, wenn R₄ und R₅ kein Wasserstoffatom darstellen,
sowie deren Salze,
- mindestens einen Kuppler der Formel: 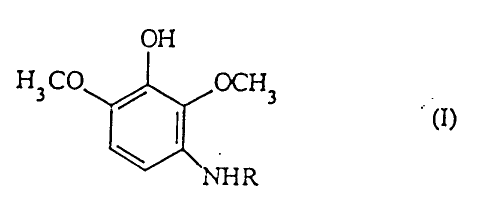 worin R Wasserstoff, eine C₁₋₄-Niedrigalkyl-, C₁₋₆-Monohydroxyalkyl-, C₂₋₆-Polyhydroxyalkyl- oder eine Alkylcarbonylgruppe darstellt, worin die Alkylgruppen 1 bis 4 Kohlenstoffatome aufweisen,
oder deren Additionssalze mit einer Säure
enthält.

2. Zusammensetzung gemäß Anspruch 1,
dadurch **gekennzeichnet**, daß
die Verbindungen der Formel (I) ausgewählt sind aus:
3-Methylamino-2,6-dimethoxyphenol,
3-Ethylamino-2,6-dimethoxyphenol,
3-Propylamino-2,6-dimethoxyphenol,
3-Butylamino-2,6-dimethoxyphenol,
3-(β-Hydroxyethyl)amino-2,6-dimethoxyphenol,
3-Acetylamino-2,6-dimethoxyphenol,
3-(β,γ-Dihydoxypropyl)amino-2,6-dimethoxyphenol und
3-Amino-2,6-dimethoxyphenol

3. Zusammensetzung gemäß einem der Ansprüche 1 und 2,
dadurch **gekennzeichnet**, daß
sie als Vorstufenverbindung eines Oxidationsfarbstoffs mindestens eine Verbindung der Formel (III) enthält, die ausgewählt ist aus:
p-Phenylendiamin, p-Toluylendiamin, Methoxy-p-phenylendiamin, Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, 2-Methyl-5-methoxy-p-phenylendiamin, 2,6-Dimethyl-5-methoxy-p-phenylendiamin, N,N-Dimethyl-p-phenylendiamin, N,N-Diethyl-p-phenylendiamin, N,N-Dipropyl-p-phenylendiamin, 3-Methyl-4-amino-N,N-diethylanilin, N,N-Di(β-hydroxyethyl)-p-phenylendiamin, 3-Methyl-4-amino-N,N-di(β-hydroxyethyl)anilin, 3-Chlor-4-amino-N,N-di(β-hydroxyethyl)anilin, 4-Amino-N,N-(ethyl,carbamylmethyl)anilin, 3-Methyl-4-amino-N,N-(ethyl,carbamylmethyl)anilin, 4-Amino-N,N-(ethyl,β-piperidinoethyl)anilin, 3-Methyl-4-amino-N,N-(ethyl,β-piperidinoethyl)anilin, 4-Amino-N,N-(ethyl,β-morpholinoethyl)anilin, 3-Methyl-4-amino-N,N-(ethyl,β-morpholinoethyl)anilin, 4-Amino-N,N-(ethyl,β-acetylaminoethyl)anilin, 4-Amino-N-(β-methoxyethyl)anilin, 3-Methyl-4-amino-N,N-(ethyl,β-acetylaminoethyl)anilin, 4-Amino-N,N-(ethyl,β-mesylaminoethyl)anilin, 3-Methyl-4-amino-N,N-(ethyl,β-mesylaminoethyl)anilin, 4-Amino-N,N-(ethyl,β-sulfoethyl)anilin, 3-Methyl-4-amino-N,N-(ethyl,β-sulfoethyl)anilin, N-((4'-Amino)phenyl)morpholin, N-((4'-Amino)phenyl)piperidin, 2-Hydroxyethyl-p-phenylendiamin, Fluor-p-phenylendiamin, Carboxy-p-phenylendiamin, Sulfo-p-phenylendiamin, 2-Isopropyl-p-phenylendiamin, 2-n-Propyl-p-phenylendiamin, 2-Hydroxy-n-propyl-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(Dihydroxypropyl)-p-phenylendiamin, N-4'-Aminophenyl-p-phenylendiamin und aus N-Phenyl-p-phenylendiamin.

4. Zusammensetzung gemäß einem der Ansprüche 1 bis 3,
dadurch **gekennzeichnet**, daß
sie als Oxidationsfarbstofr-Vorstufenverbindung vom p-Typ mindestens ein p-Aminophenol enthält, das ausgewählt ist aus: p-Aminophenol, 2-Methyl-4-aminophenol, 3-Methy-4-aminophenol, 2-Chlor-4-aminophenol, 3-Chlor-4-aminophenol, 2,6-Dimethyl-4-aminophenol, 3,5-Dimethyl-4-aminophenol, 2,5-Dimethyl-4-aminophenol, 2-Hydroxymethyl-4-aminophenol, 2-(β-Hydroxyethyl)-4-aminophenol, 2-Methoxy-4-aminophenol, 3-Methoxy-4-aminophenol, 3-(β-Hydroxyethoxy)-4-aminophenol, 2-( β-Hydroxyethoxy)methyl-4-aminophenol, 2,5-Dimethyl-4-aminophenol, 2-Methoxymethyl-4-aminophenol, 2-Ethoxymethyl-4-aminophenol, 2-Aminomethyl-4-aminophenol und aus 2-β-Hydroxyethylaminomethyl-4-aminophenol nennen.

5. Zusammensetzung gemäß einem der Ansprüche 1 bis 4,
dadurch **gekennzeichnet**, daß
sie als Vorstufenverbindung eines Oxidationsfarbstoffs mindestens ein Bisphenylenalkylendiamin der Formel (IV): 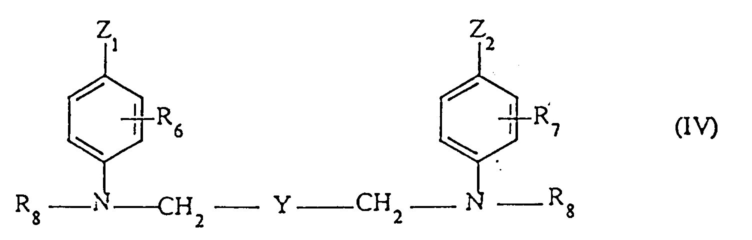 worin gilt:
Z₁ und Z₂ stellen, gleich oder verschieden, Hydroxyl- oder NHR₉-Gruppierungen dar, worin R₉ ein Wasserstoffatom oder einen Niedrigalkylrest bedeutet;
R₆ und R₇ stellen, gleich oder verschieden, entweder Wasserstoff- oder Halogenatome oder auch Alkylgruppen dar;
R₈ stellt ein Wasserstoffatom, eine Alkyl-, Hydroxyalkyl- oder Aminoalkylgruppe dar, deren Aminorest mit einer oder zwei Alkylgruppen substituiert sein kann;
Y stellt einen Rest dar, der aus den folgenden Resten ausgewählt ist:
(CH₂)ₙ-, 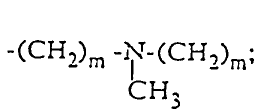 -(CH₂)ₘ-O-(CH₂)ₘ- ; -(CH₂)ₘ-CHOH-(CH₂)ₘ-
n ist eine ganze Zahl von 0 bis 8, und m ist eine ganze Zahl von 0 bis 4,
oder deren Additionssalze mit Säuren
enthält.

6. Zusammensetzung gemäß Anspruch 5,
dadurch **gekennzeichnet**, daß
sie mindestens ein Bisphenylenalkylendiamin enthält, das ausgewählt ist aus:
N,N'-bis(β-hydroxyethyl)-N,N'-bis(4'-aminophenyl)-1,3-diaminopropan-2-ol, N,N'-Bis(β-hydroxyethyl)-N,N'-bis(4'-aminophenyl)ethylendiamin, N,N'-Bis(4-aminophenyl)tetramethylendiamin, N,N'-Bis(β-hydroxyethyl)-N,N'-bis((4-aminophenyl)tetramethylendiamin, N,N'-Bis(4-methylaminophenyl)tetramethylendiamin, N,N'-Bis(ethyl)-N,N'-bis(4'-amino-3'-methylphenyl)ethylendiamin.

7. Zusammensetzung gemäß einem der Ansprüche 1 bis 6,
dadurch **gekennzeichnet**, daß
sie als Oxidationstarbstoff-Vorstufenverbindung vom o-Typ mindestens ein o-Aminophenol und/oder ein o-Phenylendiamin oder deren Additionssalze mit einer Säure enthält.

8. Zusammensetzung gemäß einem der Ansprüche 1 bis 7,
dadurch **gekennzeichnet**, daß
sie, zusätzlich, mindestens einen weiteren Kuppler enthält, der ausgewählt ist aus:
m-Diphenolen, m-Aminophenolen, die sich von denjenigen der Formel (I) unterscheiden, aus m-Phenylendiaminen, m-Acylaminophenolen, m-Ureidophenolen, m-Carbalkoxyaminophenolen, α-Naphthol, aus Kupplern mit einer aktiven Methylengruppe, wie aus β-Ketoverbindungen, Pyrazolonen oder aus heterozyklischen Kupplern, oder aus 4-, 6- oder 7-Hydroxyindol ausgewählt sind.

9. Zusammensetzung gemäß einem der Ansprüche 1 bis 8,
dadurch **gekennzeichnet**, daß
sie mindestens einen Kuppler enthält, der ausgewählt ist aus: 2,4-Dihydroxyphenoxyethanol, 2,4-Dihydroxyanisol, m-Aminophenol, Resorcin, Monomethylether von Resorcin, 2-Methylresorcin, 2-Methyl-5-N-(β-hydroxyethyl)aminophenol, 2-Methyl-5-N-(β-mesylaminoethyl)aminophenol, 6-Hydroxybenzomorpholin, 2,4-Diaminoanisol, 2,4-Diaminophenoxyethanol, 6-Aminobenzomorpholin, (2-N-(β-Hydroxyethyl)amino-4-amino)phenoxyethanol, 2-Amino-4-N, (β-hydroxyethyl)aminoanisol, (2,4-Diamino)phenyl-β,γ-dihydroxypropylether, 2,4-Diaminophenoxyethylamin, 1,3-Dimethoxy-2,4-diaminobenzol, 2-Methyl-5-aminophenol, 2,6-Dimethyl-3-aminophenol, 1-Amino-3,4-methylendioxybenzol, 1-Hydroxy-3,4-methylendioxybenzol, 2-Chlor-6-methyl-3-aminophenol, 2-Methyl-3-aminophenol, 2-Chlorresoroinol, 6-Methoxy-3-hydroxyethylaminoanilin, 1-Ethoxy-2-bis(β-hydroxyethyl)amino-4-aminobenzol, 3-Diethylaminophenol, 1,3-Dihydroxy-2-methylbenzol, 1-Hydroxy-2,4-dichlor-3-aminobenzol, 4,6-Dihydroxyethoxy-1,3-diaminobenzol, 4-Methyl-6-ethoxy-1,3-diaminobenzol, 4-Chlor-6-methyl-3-aminophenol, 6-Chlor-3-trifluorethylaminophenol, 1,3,5-Trimethoxy-2,4-diaminobenzol oder aus deren Salzen.

10. Zusammensetzung gemäß einem der Ansprüche 1 bis 9,
dadurch **gekennzeichnet**, daß
die Oxidationsfarbstoff-Vorstufenverbindungen von p- und/oder o-Typ, zusammen mit den Kupplern, 0,1 bis 7 Gew.% ausmachen, bezogen auf das Gesantgewicht der Zusammensetzung.

11. Zusammensetzung gemäß einem der Ansprüche 1 bis 10,
dadurch **gekennzeichnet**, daß
die Konzentration an Verbindungen der Formel (I) 0,05 bis 3,5 Gew.% beträgt, bezogen auf das Gesamtgewicht der Zusammensetzung.

12. Zusammensetzung gemäß einem der Ansprüche 1 bis 11,
dadurch **gekennzeichnet**, daß
sie zusätzlich einen oder mehrere Bestandteile enthält, die aus alkalisch stellenden Mitteln, oberflächenaktiven Mitteln, organischen Lösungsmitteln, Verdickungsmitteln, Antioxidantien, Eindringmitteln, Sequestriermitteln, Parfüm-Produkten und aus Puffermitteln ausgewählt sind.

13. Zusammensetzung gemäß einem der Ansprüche 1 bis 12,
dadurch **gekennzeichnet**, daß
ihr pH-Wert 4 bis 12 beträgt.

14. Verbindung der Formel (II): 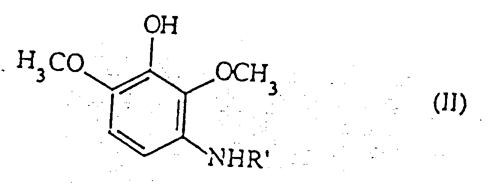 worin R' einen C₁₋₄-Alkylrest oder einen C₂- oder C₃-Mono- oder -Polyhydroxyalkylrest darstellt,
und ihre Additionssalze mit einer Säure.

15. Verbindung gemäß Anspruch 14,
ausgewählt aus 3-Methylamino-2,6-dimethoxyphenol, 3-Ethylamino-2,6-dimethoxyphenol, 3-Propylamino-2,6-dimethoxyphenol, 3-Butylamino-2,6-dimethoxyphenol, 3-(β-Hydroxyethyl)amino-2,6-dimethoxyphenol und aus 3-(β,γ-Dihydroxypropyl)amino-2,6-dimethoxyphenol.

16. Verfahren zur Färbung keratinischer Fasern,
dadurch **gekennzeichnet**, daß
man zur Anwendung bringt:
einen Bestandteil (A), enthaltend in einem zur Färbung geeigneten Milieu mindestens eine in einem der vorhergehenden Ansprüche definierte Qxidationsfarbstoff-Vorstufenverbindung vom o- und/oder p-Typ, und
einen Bestandteil (B), enthaltend in einem zur Färbung geeigneten Milieu mindestens einen Kuppler der Formel (I): 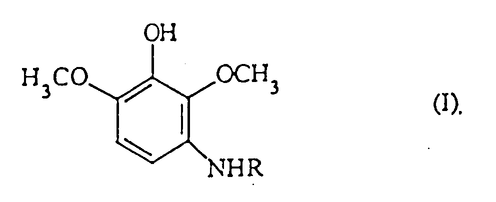 worin R Wasserstoff, eine C₁₋₄-Niedrigalkyl-, C₁₋₆-Monohydroxyalkyl-, C₂₋₆-Polyhydroxyalkyl- oder eine Alkylcarbonylgruppe darstellt, worin die Alkylgruppen 1 bis 4 Kohlenstoffatome aufweisen,
oder seine Additionssalze mit einer Säure,
und einen Bestandteil (C) aus einer oxidierenden Lösung in einer zur Entwicklung einer Färbung ausreichenden Menge,
wobei die Bestandteile (A), (B) und (C) gleichzeitig oder nacheinander angewandt werden, wobei der Bestandteil (A) vor dem Bestandteil (B) oder in Mischung mit diesem und der Bestandteil (B) vor dem Bestandteil (C) oder in Mischung mit diesem aufgebracht werden.

17. Verfahren gemäß Anspruch 16,
dadurch **gekennzeichnet**, daß
der pH-Wert der Mischung des Bestandteils (C) mit den Bestandteilen (A) und (B) oder mit dem Bestandteil (B) 3,5 bis 10 beträgt.

18. Mittel aus mehreren Bestandteilen zur Färbung keratinischer Fasern, insbesondere menschlicher Haare,
dadurch **gekennzeichnet**, daß
es umfaßt:
- einen Bestandteil (A) aus einer Zusammensetzung, die in einem zur Färbung geeigneten Milieu mindestens eine in den vorhergehenden Ansprüchen definierte Oxidationsfarbstoff-Vorstufenverbindung vom o- und/oder p-Typ enthält, und
- einen Bestandteil (B) aus einer Zusammensetzung, die in einem zur Färbung geeigneten Milieu mindestens eine Verbindung der Formel (I): 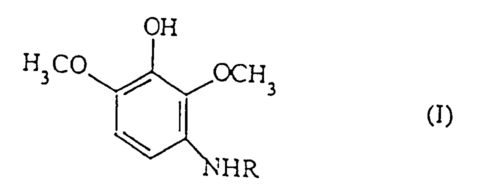 worin R Wasserstoff, eine C₁₋₄-Niedrigalkyl-, C₁₋₆-Monohydroxyalkyl-, C₂₋₆-Polyhydroxyalkyl- oder eine Alkylcarbonylgruppe darstellt, worin die Alkylgruppen 1 bis 4 Kohlenstoffatome aufweisen,
oder ihre Säureadditionssalze
als Kuppler enthält, und
- einen Bestandteil (C) aus einer oxidierenden Lösung, wobei die Bestandteile (A) und (B), jeweils der eine, in dem anderen eingeschlossen sein können.
